# EUROPEAN PATENT APPLICATION

(11) **EP 3 888 754 A1**
(43) Date of publication of application: **06.10.2021**
(21) Application number: 19891346.9
(22) Date of filing: 28.11.2019
(51) Int. Cl.: A61P 31/04, A61K 35/744, A23L 33/18, A23L 33/195, A61K 38/16

(54) **COMPOSITION FOR PROMOTING ANTIMICROBIAL PEPTIDE PRODUCTION**

(30) Priority: 29.11.2018 JP 2018223461
(71) Applicant: Megmilk Snow Brand Co. Ltd., Sapporo-shi, Hokkaido 065-0043 (JP)
(72) Inventor: KOBATAKE Eiji, Sapporo-shi, Hokkaido 065-0043 (JP); KABUKI Toshihide, Sapporo-shi, Hokkaido 065-0043 (JP)
(74) Representative: Forstmeyer, Dietmar
(86) International application number: PCT/JP2019/046527
(87) International publication number: WO 2020/111172

(57) **Abstract**

A problem to be solved by the present invention is to provide a novel material increasing antimicrobial peptide production from epithelial cells and thereby having an infection-preventing effect.

The present invention provides a composition for promoting antimicrobial peptide production containing lactic acid bacteria of the genus Lactobacillus as an active ingredient. The present invention also provides a composition for promoting antimicrobial peptide production containing a component derived from a Lactobacillus bacterial cell as an active ingredient. The present invention further provides a composition for promoting antimicrobial peptide production in which the component derived from a bacterial cell is one or more selected from the group consisting of an S-layer protein and a degradation product of the S-layer protein.

## Description

### TECHNICAL FIELD

The present invention relates to a composition for promoting antimicrobial peptide production. Particularly, the present invention relates to a composition for promoting antimicrobial peptide production containing Lactobacillus lactic acid bacteria.

### BACKGROUND ART

Antimicrobial peptides are one of defense systems possessed by living organisms and have a wide range of antimicrobial activity against bacteria, fungi, viruses, etc. Since preventive and therapeutic effects on infection can be expected by inducing the expression of the antimicrobial peptides, some solutions for increasing antimicrobial peptide production have hitherto been disclosed.

In Patent Document 1, a problem is to stimulate β-defensin production in skin cells by using non-pathogenic bacteria, and an extract containing heat-treated Lactobacillus plantarum cells and a composition containing an active fraction thereof are disclosed as a solution.

In Patent Document 2, a problem is to provide a safe antimicrobial peptide inducer having no side effect, and an antimicrobial peptide inducer containing lactic acid bacteria as an active ingredient is disclosed as a solution.

In Patent Document 3, a problem is to provide a substance inducing secretion of an antimicrobial peptide and to provide a therapeutic agent for a disease caused by a bacterium or a virus, and an antimicrobial peptide secretion inducer containing a branched-chain amino acid as an active ingredient is disclosed as a solution.

However, the solution provided by the present application is neither disclosed nor suggested in any of the documents.

### CITATION LIST

### PATENT LITERATURE

Patent Document 1: Japanese Patent No. 5224807
Patent Document 2: WO 2015/087919
Patent Document 3: Japanese Laid-Open Patent Publication No. 2003-95938

### SUMMARY OF INVENTION

### TECHNICAL PROBLEM

A problem to be solved by the present invention is to provide a novel material increasing antimicrobial peptide production from epithelial cells and thereby having an infection-preventing effect.

### SOLUTION TO PROBLEM

To solve the problem described above, the present invention includes the following configurations.
[1] A composition for promoting antimicrobial peptide production comprising: one or more selected from the group consisting of an S-layer protein that is a component derived from a Lactobacillus bacterial cell and a degradation product of the S-layer protein as an active ingredient.
[2] A composition for promoting antimicrobial peptide production comprising: a Lactobacillus helveticus cell, or a culture thereof, containing an S-layer protein that is a component derived from a Lactobacillus bacterial cell and a degradation product of the S-layer protein as an active ingredient.
[3] The composition for promoting antimicrobial peptide production according to [1] or [2], wherein the lactic acid bacteria of the genus Lactobacillus is Lactobacillus helveticus, Lactobacillus acidophilus, Lactobacillus amylovorus, or Lactobacillus buchneri.
[4] The composition for promoting antimicrobial peptide production according to any one of [1] to [3], wherein the antimicrobial peptide is β-defensin.
[5] The composition for promoting antimicrobial peptide production according to any one of [1] to [4], wherein the antimicrobial peptide production is a production of an antimicrobial peptide in epithelial cells.
[6] A food or drink for promoting antimicrobial peptide production comprising: the composition for promoting antimicrobial peptide production according to any one of [1] to [5].
[7] A food or drink for preventing infection comprising: the composition for promoting antimicrobial peptide production according to any one of [1] to [5].

### ADVANTAGEOUS EFFECTS OF INVENTION

By ingesting in a body a material inducing antimicrobial peptide production, such as S-layer protein (hereinafter sometimes referred to as SLP) derived from Lactobacillus lactic acid bacteria and a degradation product thereof, or Lactobacillus lactic acid bacterial cells containing SLP and a culture thereof, an improvement in protective ability against various infections can be expected.

### BRIEF DESCRIPTION OF DRAWINGS

[Fig. 1] Fig. 1 is a photograph showing a result of SDS-PAGE of a purified SLP.
[Fig. 2] Fig. 2 is a photograph showing results of SDS-PAGE of SLP, heated SLP, and an SLP degradation product.
[Fig. 3] Fig. 3 is a graph showing expression levels of the hBD2 gene when Lactobacillus helveticus lactic acid bacterial cells or the SLP of the lactic acid bacteria are added to Caco-2 cells.
[Fig. 4] Fig. 4 is a graph showing expression levels of the hBD2 gene when Lactobacillus helveticus SLP, heated SLP, and the SLP degradation product are added to Caco-2 cells.
[Fig. 5] Fig. 5 is a graph showing expression levels of the hBD2 gene when SLPs prepared from Lactobacillus helveticus SBT2171, Lactobacillus acidophilus JCM1132, Lactobacillus amylovorus JCM1126, and Lactobacillus buchneri JCM1115 are added to Caco-2 cells.
[Fig. 6] Fig. 6 is a graph showing expression levels of the hBD2 gene when Lactobacillus helveticus lactic acid bacterial cells or the SLP of the lactic acid bacteria are added to HSC-4 cells.

### DESCRIPTION OF EMBODIMENTS

The present invention provides a novel composition for promoting antimicrobial peptide production. The composition for promoting antimicrobial peptide production of the present invention will now be described in detail.

### (Composition for Promoting Antimicrobial Peptide Production)

S-layer protein (hereinafter sometimes referred to as SLP) is a protein found in an outermost layer of cell walls of various bacteria and is characterized by forming a regular crystal structure. The SLP is non-covalently bound to a cell wall component and is therefore extracted from bacterial cells by treatment with a chaotropic reagent such as lithium chloride. Additionally, the extracted SLP forms a regular crystal structure again when the chaotropic reagent is removed. Utilizing this characteristic enables SLP purification from bacterial cells.

Among lactic acid bacteria, it has been found that the S-layer is expressed in the genus Lactobacillus, and in the report by Mukai et al. (Japanese Journal of Lactic Acid Bacteria, 19 (1): 21-29, 2008), expression of SLP has been confirmed or estimated in 13 bacterial species including Lactobacillus-acidophilus group lactic acid bacteria. The SLPs expressed by these lactic acid bacteria have several features in common that the SLPs are basic proteins with an isoelectric point of 9 to 10 and that the SLPs have a signal peptide sequence composed of 23 to 30 amino acid residues in the N-terminal region. The SLP used in the composition for promoting antimicrobial peptide production of the present invention can be any SLP derived from the genus Lactobacillus and having such an action and is preferably Lactobacillus helveticus, Lactobacillus acidophilus, Lactobacillus amylovorus, or Lactobacillus buchneri. Regarding the Lactobacillus helveticus, SBT2171 (FERM BP-5445) is most preferable.

The SLP may not be purified from the bacterial cells and can be used as the bacterial cells themselves or culture containing the SLP; however, the purified SLP is more preferable. The bacterial cells themselves may be live bacterial cells or dead bacterial cells.

Examples of the antimicrobial peptide include the defensin family (a-defensin, β-defensin, etc.), cathelicidin (LL-37), dermcidin, LEAP-1 (hepcidin), LEAP-2, lactoferrin, Reg peptide, etc., and among them, the defensin family is preferable, and β-defensin is more preferable.

### (Method for Producing Composition for Promoting Antimicrobial Peptide Production)

The SLP is purified from lactic acid bacterial cells according to the following method. After sufficiently culturing intended Lactobacillus lactic acid bacteria in a liquid medium such as MRS liquid medium or skim milk, the bacterial cells are collected and washed as needed. The bacterial cells may be collected from colonies grown on an agar plate etc. The obtained bacterial cells are suspended and stirred directly, or after being freeze-dried, in chaotropic reagent solution, such as lithium chloride, urea, or guanidine hydrochloride, to solubilize the SLP on the bacterial cell surface. After removing solids from the solution containing the solubilized SLP, the chaotropic reagent is removed by dialysis etc. to precipitate the SLP. The precipitated SLP is collected and then washed as needed to obtain a purified SLP. The SLP used in the composition for promoting antimicrobial peptide production of the present invention may be heated because the antimicrobial peptide promoting action is maintained even when heated.

### (SLP Degradation Product)

An SLP degradation product is prepared according to the following method. Various proteases are added to a suspension of SLP at suitable concentrations and appropriately reacted. The type of protease and the degree of protease degradation are not limited. For example, conditions for degradation treatment with proteinase K are desirably 2 hours to 24 hours at 37 °C, more preferably 3 hours to 20 hours.

### (Amount of Ingestion)

Since the antimicrobial peptide production promoting action of SLP is exhibited even in the state of a degradation product, an influence on the action is probably small even if SLP is degraded by gastric acid after being orally ingested. Therefore, the SLP is desirably added to food and orally ingested. Although an amount of ingestion is not particularly limited, in the case of Lactobacillus helveticus SBT2171, it is considered that the induction of antimicrobial peptide can be expected by continuously ingesting 1 mg/day of the SLP.

### (Blending into Foods, Pharmaceuticals, and Feeds)

The purified SLP does not lose the antimicrobial peptide production promoting action due to heat treatment and therefore can be blended in foods, pharmaceuticals, and feeds by following a conventional method.

A food or drink containing the composition for promoting antimicrobial peptide production of the present invention is valuable as a food or drink for promoting antimicrobial peptide production, and by ingesting this food or drink, the antimicrobial peptide production from epithelial cells can be promoted to provide prevention from various infections, and even in the case of infection, the infection can be suppressed to a mild symptom, and quick recovery can be expected. When the composition is contained in animal feeds, the same effect can be expected in animals ingesting feeds.

A medicine containing the composition for promoting the production of the antimicrobial peptide of the present invention can be used for a disease that can be treated or prevented by promoting the production of antimicrobial peptides. Examples thereof include various infectious diseases caused by pathogens.

### (Evaluation Method)

The antimicrobial peptide production promoting action means an action of promoting the production of an antimicrobial peptide in a subject and means that the antimicrobial peptide is expressed by inducing expression of a gene for expressing the antimicrobial peptide. Therefore, in this specification, the antimicrobial peptide production promoting action and an antimicrobial peptide-inducing action are synonymously used.

The antimicrobial peptide-inducing action of the SLP of the present invention is evaluated by the method described below. The SLP or a degradation product thereof is added to cultured cells and allowed to act for a predetermined time, and an expression level of a gene encoding an antimicrobial peptide is measured. Although various cells can be used for the evaluation, epithelial cells expected to come into contact with the outside of the living body are particularly preferable. Although the antimicrobial peptide to be measured is not limited, the defensin family is preferable, and β-defensin secreted from epithelial cells is particularly preferable.

### EXAMPLES

Examples of the present invention will hereinafter be described in detail; however, the present invention is not limited thereto.

Examples of preparation of Lactobacillus bacterial cells, SLP, and an SLP degradation product used in this test will be described below.

### (Example of Preparation of Lactobacillus Lactic Acid Bacterial Cells)

After culturing Lactobacillus helveticus SBT2171 in 100 mL of MRS broth at 37 °C for 16 hours, the bacterial cells were collected by centrifugation (8,000×g, 4 °C, 10 minutes), washed twice with physiological saline and once with sterile MilliQ water, and then lyophilized (lyophilized bacterial cells).

In the following tests, lyophilized bacterial cells were used.

### (Example of Preparation of SLP)

After culturing Lactobacillus helveticus SBT2171 in 100 mL of MRS liquid medium at 37 °C for 16 hours, the bacterial cells were collected by centrifugation (8,000×g, 4 °C, 10 minutes) and washed once with sterile MilliQ water. The obtained bacterial cells were suspended in 10 mL of 1M LiCl solution containing cOmplete™ Protease Inhibitor Cocktail (Roche) and stirred at room temperature for 30 minutes. The suspension after stirring was centrifuged (10,000×g, 4 °C, 20 minutes), and a supernatant was collected. A precipitate was resuspended in 10 mL of 5M LiCl solution containing cOmplete™ Protease Inhibitor Cocktail (Roche) and stirred again at room temperature for 30 minutes. The suspension after stirring was centrifuged (10,000×g, 4 °C, 20 minutes), and a supernatant was collected. The collected supernatants were integrated, passed through a 0.2 µm filter, and then dialyzed against sterile MilliQ water with Slide-A-Lyzer G2 10K (Pierce) to precipitate SLP. The solution after dialysis was centrifuged (12,000×g, 4 °C, 20 minutes) to collect the precipitated SLP. The collected SLP was washed with 1 mL of sterile MilliQ water, suspended in 500 µL of a 1M LiCl solution and retained on ice for 15 minutes with appropriate stirring. The LiCl solution was removed by centrifugation, the SLP was washed again with 1 mL of sterile MilliQ water, resuspended in sterile MilliQ water, and lyophilized to obtain purified SLP.

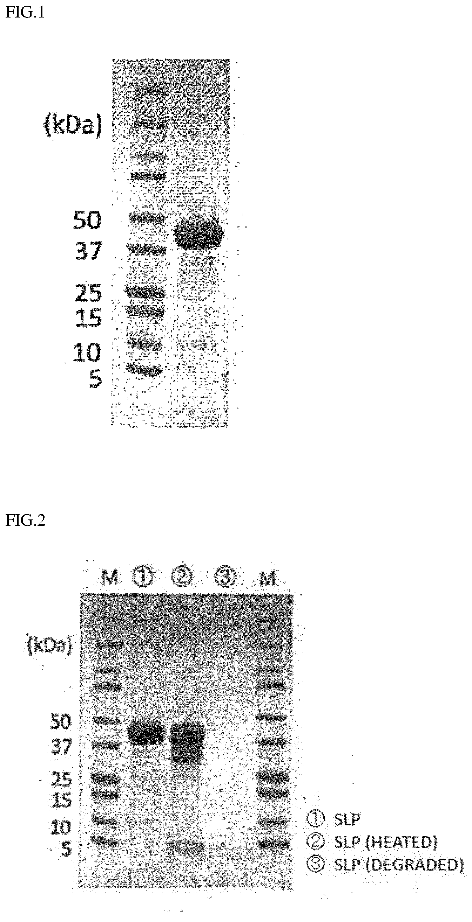

The purified SLP was confirmed by the SDS-PAGE that a band was observed in the vicinity of the target size of 43 kDa (Fig. 1). The obtained SLP was 3 mg.

### (Example of Preparation of SLP Degradation Product)

The SLP was suspended at 5 mg/mL in PBS and proteinase K (from Tritirachium album, P6556: Sigma) (10 mg/mL) was added at a final concentration of 100 µg/mL and incubated at 37 °C overnight. After the incubation, the SLP was heated at 96 °C for 10 minutes to inactivate proteinase K and the SLP degradation product was obtained. For a comparison purpose, SDS-PAGE profiles of SLP suspended in PBS, SLP suspended in PBS and heated at 96 °C for 10 minutes, and SLP decomposed by proteinase K were confirmed (Fig. 2). In the case of heated SLP, bands were observed in the vicinities of 37 kDa and 5 kDa in addition to the vicinity of 43 kDa as in the SLP. In the case of the SLP degradation product, no clear band of 5 kDa or more was observed.

### [Test Example 1] Evaluation of Antimicrobial Peptide-inducing Activity (1) Comparison with Bacterial Cells

### 1. Test Method

Subcultured Caco-2 cells (human intestinal epithelial cells) were seeded in a 12-well plate at 2.0×10⁵ cells/well and cultured overnight at 37 °C in a 5 % CO2 incubator. The cells were cultured in DMEM-high glucose (D5796: Sigma) supplemented with FBS (final conc. 10 %) (Lot. 11D264: Sigma), NEAA (final conc. 1×) (M7145: Sigma), Penicillin-Streptomycin (final conc. 100 U-100 µg/mL) (15140-122: Life technologies). After culturing, it was confirmed that the cells reached 70 to 80 % confluence, and the medium was changed to DMEM-high glucose without addition of FBS and the antibiotic (DMEM-high glucose+1×NEAA), and the cells were further cultured at 37 °C for 24 hours. Then,the medium was removed from the 12-well plate after culturing, and the cells were treated with a medium containing 10 or 50 µg/mL of Lactobacillus helveticus SBT2171 cells or the SLP purified from Lactobacillus helveticus SBT2171 for 6 hours. After the treatment with heated cells or SLP, the medium was removed and the cells were washed with PBS, and total RNA was extracted from the cells by using RNeasy Mini Kit (Qiagen). The concentration of the extracted total RNA was measured with NanoDrop 2000 (Thermo Fisher Scientific).

cDNA synthesis from total RNA was performed using the ReverTra Ace qPCR RT Master Mix with gDNA Remover (Toyobo). TakaRa PCR Thermal Cycler Dice (registered trademark) Gradient (Takara Bio) was used for the reverse transcription reaction. The obtained reverse transcription solution was used as template cDNA for real-time PCR.

Real-time PCR was carried out using Taqman (registered trademark) Fast Advanced Master Mix (Life Technologies, Cat#4444556) and TaqManGene Expression Assay (hBD2:Hs00175474_m1, GAPDH:Hs03929097_g1) (Life Technologies), and a 384-well plate (Life Technologies, Cat#4309849), and ViiA™ 7 (Life Technologies) were used. LPS was added at a concentration of 10 µg/mL as positive control, the relative gene expression level to positive control was calculated by the ΔΔCt method. The test was performed in triplicate, and the group including a sample with no amplification was defined as Not Detected (n.d.).

### 2. Test Results

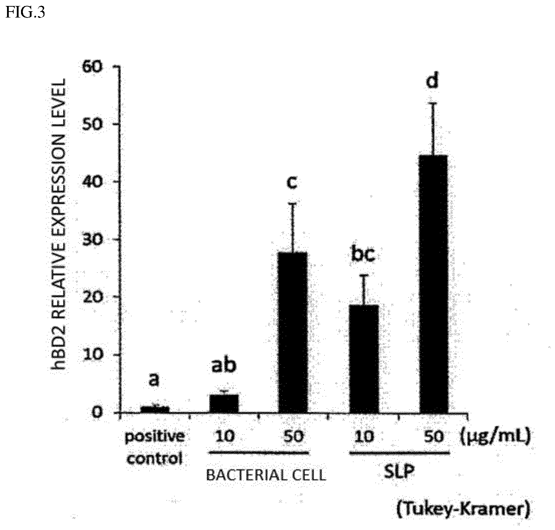

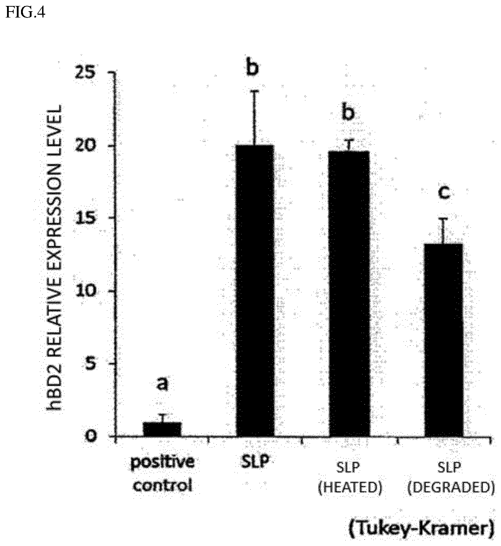

As a result of the evaluation, both SBT2171 cells and SLP increased hBD2 expression (Fig. 3). In the case of SBT2171 cells, the observed hBD2 gene expression was about 3 times as much as that of the positive control at a concentration of 10 µg/mL and about 28 times at a concentration of 50 µg/mL. On the other hand, the observed gene expression was about 19 times at a concentration of 10 µg/mL and about 45 times at a concentration of 50 µg/mL in the case of SLP. These results suggested that the antimicrobial peptide-inducing action of SLP purified from SBT2171 cells was stronger than that of SBT2171 cells. In Fig. 3, characters a, b, c, d indicate a significant difference (P<0.05) between different characters (hereinafter, the same applies to Figs. 4 to 6).

### [Test Example 2] Evaluation of Antimicrobial Peptide-Inducing Activity (2) Evaluation of SLP Degradation Product

### 1. Test Method

Caco-2 cells were seeded and the medium was changed as in "Test Example 1 (1) Comparison with Bacterial Cells" described above, subsequently, the cells were treated with SLP from Lactobacillus helveticus SBT2171, the SLP heated at 96 °C for 10 minutes, or the SLP degraded with proteinase K at a concentration of 50 µg/mL for 6 hours. The total RNA extraction, the cDNA synthesis, and the hBD2 gene expression analysis by real-time PCR were performed as described above.

### 2. Test Results

As a result, not only the untreated SLP but also the heated SLP and the degraded SLP induced hBD2 expression more strongly than positive control (Fig. 4). From this result, the purified SLP was considered to have an antimicrobial peptide-inducing action even in the state of a degraded product.

### [Test Example 3] Evaluation of Antimicrobial Peptide-Inducing Activity (3) Strain Comparison

### 1. Test method

Caco-2 cells were seeded and the medium was changed as in "Test Example 1 (1) Comparison with Bacterial Cells" described above, Subsequently, cells were treated with SLPs from Lactobacillus helveticus SBT2171, Lactobacillus acidophilus JCM1132, Lactobacillus amylovorus JCM1126, Lactobacillus buchneri JCM1115 at a concentration of 10 µg/mL for 6 hours. The total RNA extraction, the cDNA synthesis, and the hBD2 gene expression analysis by real-time PCR were performed as described above. Cells treated with no SLP were used as negative control and the relative gene expression level to positive control was also calculated.

### 2. Test Results

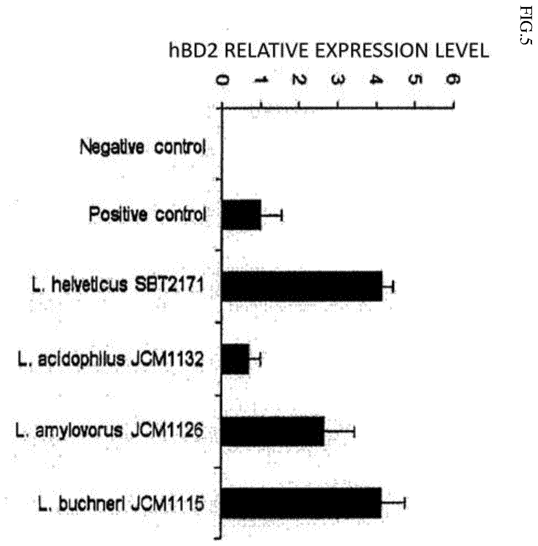

As a result, all SLPs induced hBD2 expression more than negative control. In addition, all SLPs showed high hBD2 expression equal to or stronger than that of positive control (Fig. 5). From this result, the antimicrobial peptide-inducing action caused by purified SLP was considered to be widely common in the genus Lactobacillus.

While it has been demonstrated that the molecular weight of SLP produced by lactic acid bacteria widely varies among bacterial species and that the gene structure thereof is highly-diverse even within the same bacterial species, it was surprising that the antimicrobial peptide-inducing action caused by SLP of the present invention was widely found in the genus Lactobacillus.

### [Test Example 4] Evaluation of Antimicrobial Peptide-Inducing Activity (4) Evaluation in Oral Epithelial Cells

### 1. Test method

Subcultured HSC-4 cells (human tongue epithelial cells) were seeded in a 24-well plate at 1.0×10⁵ cells/well and cultured overnight at 37 °C in a 5 % CO2 incubator. The cells were cultured in DMEM-high glucose (D5796: Sigma) supplemented with FBS (final cone. 10 %) (Lot. 42F4160K: Gibco), Penicillin-Streptomycin (final cone. 100 U-100 µg/mL) (15140-122: Life technologies). After culturing, it was confirmed that the cells reached 70 to 80 % confluence, and the medium changed to DMEM high glucose without addition of the antibiotics (DMEM-high glucose), and the cells were further cultured at 37 °C for 24 hours. Then the medium was removed from 24-well plate, and the cells were treated with medium containing 10 or 50 µg/mL of Lactobacillus helveticus SBT2171 cells or the SLP purified from Lactobacillus helveticus SBT2171 for 6 hours. Subsequently, the total RNA extraction, the cDNA synthesis, and the hBD2 gene expression analysis by real-time PCR were performed as described above.

### 2. Test Results

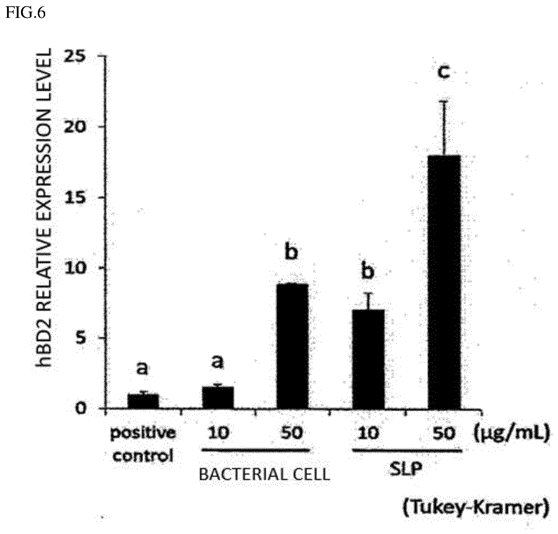

As a result, both bacterial cells and SLP increased hBD2 expression in HSC-4 cells. SLP showed high hBD2 expression more than that of bacterial cells, as in the case of Caco-2 cells (Fig. 6). These results suggested that the antimicrobial peptide-inducing action of SLP was stronger than that of bacterial cells

### (Food Formulation Example)

Thirty grams of skim milk powder, 40 g of a mixture of equal amounts of vitamin C and citric acid, 100 g of granulated sugar, and 60 g of a mixture of equal amounts of cornstarch and lactose were added to and mixed with 10 mg of SLP purified from Lactobacillus helveticus SBT2171. The mixture was packed in a stick-shaped bag to manufacture a stick-shaped health food for promoting antimicrobial peptide production of the present invention.

### (Feed Formulation Example)

Two grams of SLP purified from Lactobacillus helveticus SBT2171 was suspended in 3998 g of deionized water, heated to 40 °C, and then stirred and mixed by TK Homomixer (MARK II 160 type; manufactured by Tokushu Kika Kogyo) at 3,600 rpm for 20 minutes to obtain a 2 g/4 kg SLP solution. In 2 kg of this SLP solution, 1 kg of soybean cake, 1 kg of skim milk powder, 0.4 kg of soybean oil, 0.2 kg of corn oil, 2.3 kg of palm oil, 1 kg of corn starch, 0.9 kg of wheat flour, 0.2 kg of bran, 0.5 kg of vitamin mixture, 0.3 kg of cellulose and 0.2 kg of a mineral mixture were blended and sterilized by heating at 120 °C for 4 minutes to manufacture 10 kg of a feed for promoting antimicrobial peptide production of the present invention.

### (Pharmaceutical Formulation Example)

A liquid culture of Lactobacillus helveticus SBT2171 was centrifuged at 7,000 rpm for 15 minutes at 4 °C, and then washed with sterile water and centrifuged three times to obtain washed bacterial cells. The washed bacterial cells were lyophilized to obtain bacterial cell powder. Four parts of skim milk powder were mixed with 1 part of this bacterial cell powder, and 1 g of this mixed powder was tableted by a conventional method with a tableting machine to prepare each tablet for promoting antimicrobial peptide production of the present invention.

### INDUSTRIAL APPLICABILITY

By ingesting in a body a material inducing antimicrobial peptide production, such as SLP derived from Lactobacillus lactic acid bacteria and a degradation product thereof, or Lactobacillus lactic acid bacterial cells containing SLP and a culture thereof, an improvement in protective ability against various infections can be expected.

## Claims

1. A composition for promoting antimicrobial peptide production comprising: one or more selected from the group consisting of an S-layer protein that is a component derived from a Lactobacillus bacterial cell and a degradation product of the S-layer protein as an active ingredient.

2. A composition for promoting antimicrobial peptide production comprising: a Lactobacillus helveticus cell, or a culture thereof, containing an S-layer protein that is a component derived from a Lactobacillus bacterial cell and a degradation product of the S-layer protein as an active ingredient.

3. The composition for promoting antimicrobial peptide production according to claim 1 or 2, wherein the lactic acid bacteria of the genus Lactobacillus is Lactobacillus helveticus, Lactobacillus acidophilus, Lactobacillus amylovorus, or Lactobacillus buchneri.

4. The composition for promoting antimicrobial peptide production according to any one of claims 1 to 3, wherein the antimicrobial peptide is β-defensin.

5. The composition for promoting antimicrobial peptide production according to any one of claims 1 to 4, wherein the antimicrobial peptide production is a production of an antimicrobial peptide in epithelial cells.

6. A food or drink for promoting antimicrobial peptide production comprising: the composition for promoting antimicrobial peptide production according to any one of claims 1 to 5.

7. A food or drink for preventing infection comprising: the composition for promoting antimicrobial peptide production according to any one of claims 1 to 5.
